# EUROPEAN PATENT APPLICATION

(11) **EP 4 616 834 A1**
(43) Date of publication of application: **17.09.2025**
(21) Application number: 24184071.9
(22) Date of filing: 24.06.2024
(51) Int. Cl.: A61F 13/534, A61F 13/551, A61F 13/56

(54) **FOLDED ABSORBENT ARTICLE**

(30) Priority: 14.03.2024 EP 24163641
(71) Applicant: Ontex BV, 9255 Buggenhout (BE); Ontex Group NV, 9320 Erembodegem (BE)
(72) Inventor: Staelens, Eva, 9900 Eeklo (BE); Descheemaecker, Evan, 9930 Zomergem (BE)
(74) Representative: Saurat, Thibault

(57) **Abstract**

The present disclosure pertains to an assembly (1) comprising a release paper (22) and an absorbent article (10), the absorbent article (10) comprising :
- a topsheet (14),
- a backsheet (16),
- an absorbent core (12) arranged between said topsheet (14) and backsheet (16), and
- a layer of adhesive (20) being arranged on a side of the backsheet (16) opposite to the absorbent core (12),

the release paper (22) being arranged on a side of the layer of adhesive (20) opposite to the backsheet (16),
the release paper (22) and the absorbent article (10) extending in respective longitudinal, transversal and vertical directions (L,T,V). According to the present disclosure, the absorbent article (10) is maintained in a folded configuration by the release paper (22) and the longitudinal direction of the release paper (22) is orthogonal to the longitudinal direction of the absorbent article (10).

## Description

### TECHNICAL FIELD

The disclosure pertains to the technical field of absorbent articles for hygiene products and the packaging of such absorbent articles. In particular, the present disclosure relates to individually packaged absorbent articles selected from sanitary napkins, adult incontinence pads or panty liners, which are configured to collect and contain blood, menses, urine, vaginal fluids and avoid leakage. The present disclosure also pertains to the packaging process to wrap or enclose such absorbent articles.

### BACKGROUND

Absorbent articles selected from sanitary napkins, adult incontinence pads or pantyliners, used to collect vaginal discharges are well known in the art. Typically, these absorbent articles commonly comprise a topsheet, a backsheet and an absorbent core arranged in-between said topsheet and backsheet as well as a siliconized release paper protecting the adhesive arranged on the backsheet. These articles are typically individually enclosed, or packaged, in a wrapper also commonly called a pouch or wrapping pouch. These absorbent articles are sold in a folded configuration where the absorbent article is folded in two, three or even four with the wrapper securing this folding. These wrappers or pouches are made out of plastic films such as a polypropylene or polyethylene films since these material have several beneficial physical properties such as flexibility, deformability, *etc.* as well as for cost reasons. However, these wrappers are always made from petroleum-derived materials and thus are not environment-friendly.

For example publications such as EP 0 750 896 A2, US6,186,993 B2 or EP 2 532 333 A1 show these conventional pouches where the article is folded multiple times and the packaging wrapper, usually made of polyolefin material, maintains that article folded.

There is still a need to improve the environmental impact of these wrappers and offer a solution that involves less plastic without degrading the performance of the packaging of these absorbent articles.

The invention thereto aims to provide an environmentally friendly packaging for absorbent articles selected from sanitary napkins, adult incontinence pads or pantyliners.

### SUMMARY OF THE DISCLOSURE

The present disclosure relates to an assembly comprising a release paper and an absorbent article, the absorbent article comprising :
- a topsheet,
- a backsheet,
- an absorbent core arranged between said topsheet and backsheet, and
- a layer of adhesive being arranged on a side of the backsheet opposite to the absorbent core,

the release paper being arranged on a side of the layer of adhesive opposite to the backsheet,
the release paper and the absorbent article extending in respective longitudinal, transversal and vertical directions,
wherein the absorbent article is maintained in a folded configuration by the release paper and the longitudinal direction of the release paper is orthogonal to the longitudinal direction of the absorbent article.

For sake of clarity, for any given element such as an absorbent article or a release paper, the longitudinal direction (L) corresponds to the dimension defining the length, the transversal direction (T) corresponds to the dimension defining the width, the vertical direction (V) corresponds to the dimension defining the height. For any element such as an absorbent article or a release paper, the length corresponds to the dimension extending in the longitudinal direction and is longer than the width, said width being the dimension extending in the transversal direction and said height being the dimension extending in the vertical direction. This is more apparent when the element is in an unfolded configuration.

Preferably the absorbent article is selected from a sanitary napkin, a pantyliner or an incontinence pad. More preferably, the absorbent article is selected from a sanitary napkin, a pantyliner or an incontinence pad free of an individual wrapping pouch.

In other words, the release paper and the absorbent article each have a length, width and height. The length corresponds to the greatest dimension when compared to the width and height and extends and defines a longitudinal direction for each element. The release paper and the absorbent article both extend in a three-dimensional space defined by the longitudinal, transversal and vertical axis with each release paper and the absorbent article comprising directional vectors, namely longitudinal, transversal and vertical vectors, with the longitudinal vector of the release paper being orthogonal, or perpendicular, to the longitudinal vector of the absorbent article. For sake of clarity, the absorbent article can be either in a partially folded configuration, a fully folded configuration and in an unfolded configuration, in any of these configurations the absorbent article extends in longitudinal, transversal and vertical directions (L,T,V).

The absorbent article is arranged in a folded configuration such that absorbent article is maintained directly or indirectly in said folded configuration by the release paper. By arranging the absorbent article, or the absorbent article, in a way that the release paper can maintain said absorbent article in a folded configuration thereby removing the need altogether for wrapper, the environmental impact of the packaging is lowered since less material is used.

According to an embodiment, the absorbent article extends in longitudinal, transversal and vertical directions, and comprises in an unfolded configuration with respect to the longitudinal direction, a front portion, a rear portion and a central portion arranged between said front and rear portions, when maintained in said folded configuration, the front and rear portions are folded onto the central portion.

According to an embodiment, the central portion is defined by the length of the wings taken in the point in which they extend from the longitudinal sides of the absorbent article in a direction parallel to the longitudinal axis.

According to an embodiment, when in said folded configuration, the front and rear portions are folded onto the central portion and/or the wings, the front and rear portions overlaying the central portion and/or the wings with respect to the vertical direction.

According to an embodiment, the absorbent article comprises wings, wherein each wing comprises an area of adhesive arranged on the side of the backsheet opposite to the absorbent core.

According to an embodiment, a layer of adhesive comprises a first area of adhesive, said first area of adhesive being applied in a discontinuous pattern with a first sub-area of adhesive arranged in the front portion, a second sub-area of adhesive arranged in the central portion and a third sub-area of adhesive arranged in the rear portion, said first, second and third sub-area of adhesive being distinct from one another.

According to an embodiment, the release paper comprises an overlapping region, where the two longitudinal extremities of the release paper are overlapping with adhesive material being arranged in said overlapping region between said two longitudinal extremities of the release paper.

For sake of clarity, it is inherent that a release paper extending in respective longitudinal, transversal and vertical directions will have a length, hence two opposite longitudinal extremities, in other words, the release paper comprises a first and a second longitudinal extremities.

According to an embodiment, a gap is arranged between the two longitudinal extremities of the release paper.

According to an embodiment, wherein in a folded configuration the surface are of the folded release paper covers between 50 and 99 % of the surface area of the folded absorbent article.

According to an embodiment, the release paper is partially coated by a peeling agent, such that the surface area of peeling agent is comprised between 40% and 95%, preferably between 50% and 90% of the total area of a surface of the release paper.

According to an embodiment, the ratio between the total surface area of peeling agent arranged on the release paper and the total surface area of adhesive arranged on the backsheet is comprised between 1 and 1.5, preferably between 1 and 1.2, for sake of clarity, the value 1 is included in said range. For example, the area, or the surface area, of peeling agent on the release paper only overlaps the area, or the surface area, of adhesive on the backsheet. In other words, the peeling agent coated on the release paper and the adhesive on the backsheet are congruent, meaning are of same size and shape and are placed one over the other and exactly matching. Similarly, the release paper and the adhesive on the backsheet can be congruent, meaning are of same size and shape and are placed one over the other and exactly matching.

The present disclosure also pertains to a method for manufacturing an assembly comprising a release paper and an absorbent article according to any of the preceding claims, wherein said method comprises the following steps :
- providing said absorbent article and said release paper;
- applying a release tape onto the front and/or rear portion to cover the first and/or third sub-area of adhesive;
- applying the release paper in a direction orthogonal to the longitudinal direction of the absorbent article with a first longitudinal extremity of the release paper being applied onto a first portion of the second sub-area of adhesive;
- folding the front portion onto the central portion and folding the rear portion onto the front portion or folding the rear portion onto the central portion and folding the front portion onto the rear portion;
- optionally folding the wings onto the folded front and rear portions;
- wrapping the release paper around the folded absorbent article onto the folded front and rear portion or optionally onto the folded wings; and
- securing the release paper to the second portion of the second sub-area of adhesive with the second longitudinal extremity of the release paper being applied onto the second portion of the second sub-area (40c) of adhesive.

According to an embodiment, said method comprises the following steps :
- providing an absorbent article and said release paper;
- optionally folding the wings onto the central portion and applying a release liner to keep the wings folded onto the central portion;
- folding the front portion onto the central portion;
- applying a release paper in a direction orthogonal to the longitudinal direction of the absorbent article with a first longitudinal extremity of the release paper being applied onto the first sub-area of adhesive;
- folding the rear portion onto the release paper;
- wrapping the release paper around the folded absorbent article and securing the release paper onto the second sub-area of adhesive ; and
- securing the release paper to the third sub-area of adhesive with the second longitudinal extremity of the release paper being applied onto the third sub-area of adhesive.

According to an embodiment, wherein the absorbent article comprises wings, said method comprises a further step of applying a release liner onto second area of adhesive arranged on the wings.

The present disclosure also pertains to a container comprising a plurality of folded absorbent articles as described herein. Said container comprising a plurality of assemblies as described herein, said container being selected from a reusable bag, a paper bag, a cardboard box or a metal box.

While these absorbent articles use less material and thus are environmentally friendly, conventional pouches are also serves as a protection against contaminants and moisture for the absorbent article. With such folding, this protection is slightly lowered. While it is not necessary to have such protection when staying at home, absorbent articles being sold in cardboard boxes or plastic bags, a user can leave them in their package and grab one when needed, however when traveling it can be beneficial to have a reusable container such as a small bag, a paperbag, a cardboard box or a metal box to carry a limited number of absorbent article, e.g. one to five absorbent articles. The reusable container can be resealable. Hence the present disclosure also pertains to a container comprising a plurality of assemblies comprising a folded absorbent article and a release paper as described herein. The container is preferably dimensioned to carry a limited number of absorbent articles such as one, two, three, four or five absorbent articles. The present disclosure can also pertain to containers containing as much as 10, 20 or even more absorbent articles, for example when traveling on a trip of a longer duration, e.g. a three-month long trip.

According to an embodiment, the method further comprises an additional step of printing a graphic element on said backsheet, release tape, release paper or release liner.

All of these embodiments mentioned above can be taken individually or in combination. Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**Fig. 1** illustrates a schematic cross section of an absorbent article;
**Fig. 2** shows a schematic bottom view of a sanitary napkin;
**Fig. 3** depicts a schematic perspective view of an absorbent article according to an embodiment;
**Fig. 4** represents a schematic perspective view of an absorbent article according to another embodiment;
**Fig. 5** shows a schematic perspective view of an absorbent article according to another embodiment;
**Fig. 6** (6A to 6I) depicts a schematic view of a method of folding an absorbent article;
**Fig. 7** (7A to 7G) depicts a schematic view of a method of folding an absorbent article;
**FIG. 8** shows a schematic bottom view of a pantyliner or incontinence pad;
**FIG. 9** shows a schematic top view of an assembly comprising an absorbent article and a release paper in an unfolded configuration.

### DETAILED DESCRIPTION OF THE INVENTION

The present invention concerns the packaging of individually packaged absorbent articles selected from sanitary napkins, adult incontinence pads or pantyliners.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The recitation of numerical ranges by endpoints includes all numbers and fractions subsumed within that range, as well as the recited endpoints.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

"Absorbent article" refers to devices that absorb and contain liquid, and more specifically, refers to devices that are placed against or in proximity to the body of the wearer to absorb and contain the various exudates discharged from the body. Absorbent articles preferably comprise a longitudinal axis defining a length and a transversal axis defining a width, said transversal axis being perpendicular to said longitudinal axis as well as a vertical axis defining a height or thickness. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious topsheet, a backsheet joined to the topsheet, and an absorbent core positioned and held between the topsheet and the backsheet. The topsheet is operatively permeable to the liquids that are intended to be held or stored by the absorbent article, and the backsheet may or may not be substantially impervious or otherwise operatively impermeable to the intended liquids. The absorbent article may also include other components, such as acquisition distribution layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface. More particularly, the present disclosure pertains to absorbent articles in relation to feminine hygiene, or more specifically individually packaged absorbent articles selected from sanitary napkins, adult incontinence pads or panty liners, which are configured to collect and contain blood, menses, urine, vaginal fluids and avoid leakage.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common in feminine hygiene articles to combine cellulosic fluff pulp with superabsorbent polymers in an absorbent material or to use cellulosic fluff pulp alone.

"Acquisition distribution layer", "ADL" or "surge management portion" refers to a sub-layer which preferably is a nonwoven wicking layer arranged directly under the topsheet of an absorbent product, which speeds up the transport and improves distribution of fluids throughout the absorbent core. The surge management portion has the ability to quickly collect and temporarily hold liquid surges, and to transport the liquid from its initial entrance point to other parts of the absorbent structure, particularly the retention portion. This configuration can help prevent the exudates from pooling and collecting on the portion of the absorbent garment positioned against the wearer's skin.

The term "adhesive" as used herein is intended to refer to any suitable hot melt, water or solvent borne adhesive that can be applied to a surface of a film layer in the required pattern or network of adhesive areas to form the film-nonwoven laminate of the present disclosure. Accordingly, suitable adhesives include conventional hot melt adhesives, pressure-sensitive adhesives and reactive adhesives (i.e., polyurethane). The absorbent article can comprise different kinds of adhesive. For example, the absorbent article can comprise hot melt adhesive for bonding the topsheet to the backsheet and non-structural or pressure sensitive adhesive for bonding the absorbent article to the garment. For example, the absorbent article can comprise mechanical bonding such as ultrasonic or hydroentanglement for bonding the topsheet to the backsheet and non-structural or pressure sensitive adhesive for bonding the absorbent article to the garment.

As used herein, the terms "non-structural adhesive" or "pressure sensitive adhesive" refers to any adhesive with a low bonding strength usually demonstrating a load-carrying capacity of less than 1000 pound-force per square inch (psi). Such non-structural adhesives comprise for example vinyls, polychloropene, styrene block copolymer and polyurethane.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which topsheet is directly joined to backsheet by affixing topsheet directly to backsheet, and configurations wherein topsheet is joined to backsheet by affixing topsheet to intermediate members which in turn are affixed to backsheet. Topsheet and backsheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet to backsheet. It should be readily appreciated that the above-described attachment means may also be employed to interconnect and assemble together the various other component parts of the article described herein.

The term "backsheet" or "backsheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing through. Examples of breathable backsheet materials are porous polymeric films, nonwoven laminates of spunbond and meltblown layers and laminates of porous polymeric films and nonwoven materials or a viscose or cotton nonwoven that is coated with a hydrophobic or liquid repellent coating.

As used herein, the term "basis weight" refers to an average weight, or a range of weight, of the component in question, namely the layer of cellulosic fibers or the layer of peeling agent, per square meter. Term such as "grammage" is equivalent to "basis weight" and is expressed in grams per square meter, g/m² or gsm.

For instance, the basis weight of the layer of cellulosic fibers is preferably measured according to the ISO 536 standard or any standard method. The amount of peeling agent or sealing agent on the wrapper can be measured by taking a sample of the wrapper where the peeling agent or the sealing agent is arranged, weighing said sample before and after removal of the peeling agent or sealing agent (in grams) the difference giving the weight of the peeling agent or sealing agent, and dividing the weight of peeling agent or sealing agent by the area of the sample (in m²) thereby obtaining the amount of peeling agent or sealing agent in gsm.

As used herein, the term "body-facing", "skin-facing" or "bodyside" side or surface means that surface of the article or component which is intended to be disposed toward or placed adjacent to the body of the wearer during ordinary use, while the "outward", "outward-facing" or "garment-facing" side or surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

As used herein, the term "cellulosic" or "cellulosic fibers" is meant to include any material having cellulose as a major constituent, and specifically comprising at least 50 percent by weight cellulose or a cellulose derivative. Thus, the term includes cotton, typical wood pulps, nonwoody cellulosic fibers, kraft paper, cellulose acetate, cellulose triacetate, rayon, thermomechanical wood pulp, chemical wood pulp, debonded chemical wood pulp, milkweed, or bacterial cellulose.

As used herein "configuration", "conformation", "state" are synonymous and interchangeable and means an arrangement of an element, such as the absorbent article, in a particular form, figure, or combination.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "garment" means any type of apparel which may be worn. This includes diapers, training pants, incontinence products, surgical gowns, industrial workwear and coveralls, undergarments, pants, shirts, jackets and the like.

The term "graphic" or "graphic element" includes, but is not limited to, any type of design, image, mark, figure, codes, words, patterns, or the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", "bond", "bonded", "affixed", "associated", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone.

The term "longitudinal", "transversal" and "vertical" as used herein are with respect to the assembly, comprising the absorbent article and release paper, in an assembled state or in a disassembled or partially assembled state and/or in an unfolded state or in a fully or partially folded state. In other words, the assembly defines the longitudinal, transversal and vertical directions. The term "in the direction" as used herein means along that direction, e.g. in the longitudinal direction means along the longitudinal direction. With reference to the release paper or absorbent article, a length corresponds to the dimension extending in the longitudinal direction and is preferably longer than the width, said width being the dimension extending in the transversal direction. The terms "top", "bottom", "upper" and "lower" are all in reference to said vertical direction.

All length, width, and height or thickness can measured with a caliper preferably a micrometer caliper gauge by taking the largest distance separating two points along one direction as defined herein for a given element, e.g. the sanitary napkin or release paper in in a folded, unfolded or partially folded state, or in other words, measuring the distance between two edges of said element along one direction.

The term "length", "width" and "thickness or height" as used herein are respectively in reference to the longitudinal, transversal and vertical direction as defined herein.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layers or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "peeling treatment" as used herein, namely in the context of the release paper and/or release liner and/or release tape, means a peeling treatment applied to the peelable paper or liner or tape where a peeling agent, preferably selected from and not limited to silicon resin series, fluororesin series, octadecilisocyanate series or the like is applied, or coated, onto said paper or liner or tape. It is preferable that the peeling treatment is carried out by applying a coat of silicon resin series as the peeling agent material. The peeling agent is dried by heating or by irradiating ultraviolet rays or the like so as to be polymerized, or by spraying it, to thereby form a thin coat or thin layer of peeling agent. Using a thin coat of silicone as the peeling agent is preferable since silicone corresponds to a material that has heat-resistant properties, thus more stable in industrial processes, as well as antimicrobial properties which is better to ensure the protection of the absorbent article from contamination prior to use. The silicone has other properties such as water repellent and is greaseproof. The wrapper 22 comprises a layer of peeling agent 32 comprising a basis weight comprised between 0,3 gsm and 3 gsm, preferably between 0,5 gsm and 1 gsm.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

The term "portion" is used in its general sense meaning a part or share of something larger. For example, in reference to the front, central or rear portions, these portions can be considered as parts or regions of the absorbent article. In reference to a portion of sub-area of adhesive, this portion can be considered as a part or share or region of that entire sub-area of adhesive.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood. The material is typically lightweight and has absorbent properties.

By "channels" or "embossments", it is meant that the structure referred to (e.g. the absorbent core) comprises recessed regions forming visible conduits or passages typically extending along the longitudinal axis of the core and having a depth in a direction perpendicular to said longitudinal axis. By "visible" it is herein intended clearly visible by naked eye and typically that the channels have a width generally greater than 1mm, preferably from 5mm to 50 mm, more preferably from 8mm to 40mm, more preferably from 10mm to 30mm, even more preferably from greater than 10mm to less than 25mm.

By "substantially", it is meant at least the majority of the structure referred to. Use of the term "substrate" includes, but is not limited to, woven or nonwoven webs, porous films, ink permeable films, cellulosic pulp, paper, composite structures, or the like.

Superabsorbent materials suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. The topsheet fabrics may be composed of a substantially hydrophobic material, and the hydrophobic material may optionally be treated with a surfactant or otherwise processed to impart a desired level of wettability and hydrophilicity.

The term "release liner", "release tape" and "release paper" are equivalent as used herein, these terms all mean a removable paper layer that is coated with peeling agent, such as silicone and arranged to cover or protect a layer of adhesive. For sake of clarity and to distinguish one term over the other "release liner" is in reference to the wings exclusively whereas "release paper" is in reference to the main body region and eventually wings and whereas "release tape" is in reference to the main body region exclusively, namely to the front and/or rear portions of the main body region.

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

Embodiments according to the disclosure will now be described. It is understood that technical features described in one or more embodiments maybe combined with one or more other embodiments without departing from the intention of the disclosure and without generalization therefrom.

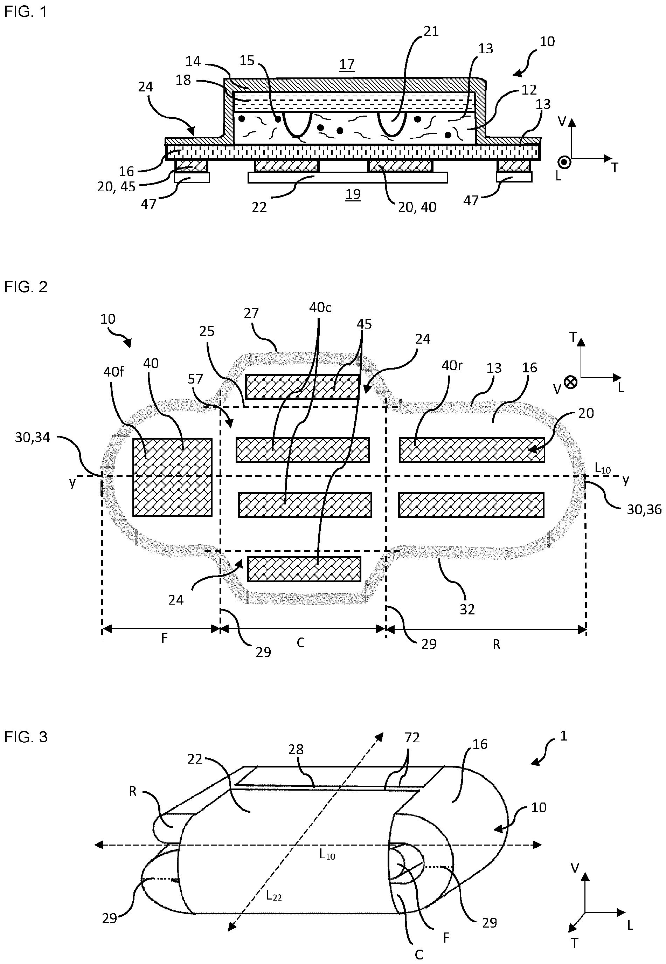

As illustrated in Fig.1, an absorbent article 10 according to the present disclosure, such as a sanitary napkin 10, or sanitary towel, comprises an absorbent core 12 arranged between a liquid permeable topsheet 14 and a liquid impermeable backsheet 16. An optional acquisition distribution layer 18, or surge layer, also called ADL 18, can be positioned between said topsheet 14 and said absorbent core 12. The absorbent core 12 comprises absorbent material. The backsheet 16 is joined to the topsheet 14 with the absorbent core 12, and the optional ADL 18, being positioned and enclosed between the topsheet 14 and the backsheet 16. As illustrated in Fig. 1, the topsheet 14 and the backsheet 16 are associated with one another. The topsheet 14 and backsheet 16 are associated, or joined at the periphery of the sanitary napkin 10 as illustrated in FIG. 2 in bonding regions 13 by attachment means such as an adhesive, mechanical bonds, sonic bonds, thermal bonds, in other words, the bonding regions 13 define the outline or perimeter of the absorbent article 10. The topsheet 14 is arranged on the body-facing side 17 whereas the backsheet 16 is arranged on the garment-facing side 19 of the absorbent article 10. Optionally, the absorbent core 12 and/or the optional ADL 18, can comprise at least one channel 21 or at least one embossment 21. As illustrated in FIG. 1, the absorbent core 12 comprises two channels 21 or two embossments 21. The topsheet 14 and ADL 18 can be embossed as well, separately or together with the absorbent core 12. For example, the absorbent article 10 can comprise a topsheet 14 and ADL 18 embossed and a non-embossed absorbent core 12.

The absorbent article 10 according to the present disclosure is selected from a pantiliner, a sanitary napkin or an incontinence pad and can comprise wings or be wingless, namely pantyliners and incontinence pads are wingless or free of wings whereas sanitary napkins comprise wings or flaps.

The absorbent article 10 comprises on the garment-facing side 19 of the backsheet 16 at least one layer of adhesive 20 so that the absorbent article 10 can adhere onto a garment or undergarment, said at least one layer of adhesive 20 ensuring that the absorbent article 10 stays fastened and secured on the underwear. In order to maintain the structural integrity of the adhesive before being used, the absorbent article 10 comprises a release paper 22 corresponding to a peelable layer destined to protect the at least one layer of adhesive 20. The release paper 22 is preferably a siliconized paper acting as a release liner. In other words, the release paper 22 comprises a paper layer comprising cellulosic fibers, meaning paper, and a peeling layer or coating comprising a peeling agent selected from silicon resin series, fluororesin series, octadecilisocyanate series. In other words, the release paper 22 is a paper layer coated with a silicone derivative. According to an embodiment, the peeling layer comprises a basis weight comprised between 0,3 gsm and 3 gsm, preferably between 0,5 gsm and 1 gsm and the paper layer comprises a basis weight comprised between 10 gsm and 80 gsm, preferably between 15 and 60 gsm. The release paper 22 can be recyclable. The adhesive material, meaning the layer of adhesive 20, can be applied in bloc and/or in stripes and/or in dots and/or in swirls. The stripes can be elongated in the longitudinal direction and/or in the transversal direction. The at least one layer of adhesive 20 is applied in a pattern that is described hereunder.

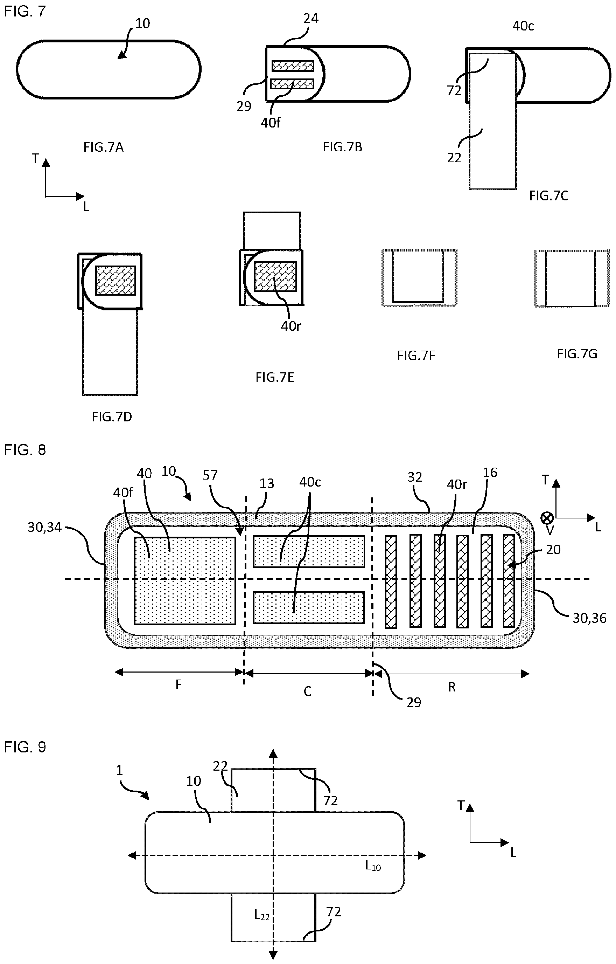

An absorbent article 10, and more particularly a sanitary napkin, is illustrated in FIG. 2 in an unfolded configuration, e.g. laid flat on a surface. Similarly, an absorbent article 10, and more particularly a pantyliner or incontinence pad, is illustrated in FIG. 8 in an unfolded configuration, e.g. laid flat on a surface. In both illustration, the absorbent article 10 extends in longitudinal, transversal and vertical directions L, T, V as illustrated in FIG. 1 and 2, thereby defining a length in the longitudinal direction L, a width W in the transversal direction T and a thickness or height in the vertical direction V. In an unfolded configuration as shown in FIG. 2, the length of the absorbent article 10 is greater than the width. The longitudinal axis is hereby chosen in the front-to-back direction of the absorbent article 10 when referring to said article being worn, and the transversal axis is conventionally chosen in the left-to-right direction of the absorbent article 10 when referring to said article being worn.

As explained above, figure 2 illustrates an absorbent article 10 selected from a sanitary napkin. A sanitary napkin 10 comprises a topsheet 14, a backsheet 16, an absorbent core 12 arranged between said topsheet 14 and backsheet 16, and two foldable wings 24 or flaps 24 arranged at opposite sides of the sanitary napkin 10 with respect to the transversal direction T. As illustrated in FIG. 2, the sanitary napkin 10 extends in longitudinal, transversal and vertical directions L,T,V, the sanitary napkin 10 has a longitudinal centerline y-y, extending in the longitudinal direction L₁₀, a pair of transverse sides 30, meaning a pair of sides arranged at each longitudinal ends or longitudinal extremities, of the sanitary napkin 10, and a pair of longitudinal sides 32 extending therebetween, meaning a pair of sides arranged along each transversal ends of the sanitary napkin 10 and connecting the transverse sides 30 to one another. Similarly, the pair of transverse sides 30 connects the two longitudinal sides 32 to one another. In other words, the sanitary napkin 10 comprises two ends, or extremities, at least partially extending in the transversal direction T, *i.e.* the transversal sides 30, and two ends at least partially extending in the longitudinal direction L₁₀, i.e. the longitudinal sides 32. As illustrated in FIG. 2, the transversal sides 30 are arcuate whereas the longitudinal sides 32 are linear. The sanitary napkin 10 has a front edge 34 and a rear edge 36 positioned on opposite apexes of said transverse sides 30, meaning at each longitudinal extremities of the sanitary napkin 10. The sanitary napkin 10 is divided with respect to the longitudinal direction L, into three sections or portions, a front portion F, a rear portion R and a central portion C arranged between said front and rear portions F,R.

The sanitary napkin 10 also comprises wings 24, or flaps 24, arranged at each transversal end of the sanitary napkin 10, the wings 24 extend transversely beyond the longitudinal sides 32 of the sanitary napkin 10. In other words, the flaps 24 are transversally, or laterally, outboard of the longitudinal centerline y-y and central portion C of the sanitary napkin 10. In other terms, each flap 24 comprises a proximal end 25 and a distal end 27 with respect to the longitudinal centerline y-y and with respect to the transversal direction T. Said proximal end 25 is connected to the sanitary napkin 10, *i.e.* connecting the sanitary napkin 10 and the wing 24 and said distal end 27 is the point furthest from the longitudinal centerline y-y. As used herein the term "central portion" C refers to the portion of the sanitary napkin 10 arranged in between the front and rear portions F, R and in between the proximal ends 25 of the flaps 24, meaning intermediate, particularly laterally intermediate of the two wings 24. In other words, the central portion C is defined by the length of the wings 24 taken in the point in which they extend from the longitudinal sides 32 of the sanitary napkin 10 in a direction parallel to the longitudinal axis y-y. In other words, the central portion C is delimited longitudinally by the front and rear portions F, R and transversally by the proximal ends 25 of the flaps 24. The wings 24 may be comprised of an integral and contiguous extension of the topsheet 14, the backsheet 16, or a laminate of both. Alternatively, the wings 24 may be made of separate and independent pieces of material joined to the longitudinal sides 32 of the sanitary napkin 10. Each wing 24 has one face generally coextensive of the topsheet 14 and a mutually opposed face generally coextensive of the backsheet 16.

As illustrated in FIG. 2, the sanitary napkin 10 comprises two regions, a main body 57 and the wings 24. The main body 57 is the region, or portion of the absorbent article 10 comprising the absorbent core 12 and the eventual ADL 18 and is oblong or oblong-shaped, meaning comprising a shape, e.g. a rectangular, that is longer than it is wide and whose corners are rounded. The wings 24 correspond to rectangular or trapezoidal extensions outboard of the main body 57, meaning outboard of the oblong shape, with respect to the transversal direction T. The wings 24 are inboard of the main body 57 with respect to the longitudinal direction L and are arranged in the central portion C of the sanitary napkin 10 with respect to the longitudinal direction L. The main body 57 can be rectangular or trapezoidal. The wings 24, only comprising a laminate of topsheet 14 and backsheet 16, are flexible and foldable, meaning the wings 24 can be easily pivoted or rotated at least around a longitudinal axis. The topsheet 14 and the backsheet 16 are associated with one another along the periphery of the sanitary napkin 10 at the main body 57 region and at the wings 24 in bonding regions 13. The pair of longitudinal sides 32 are in reference to the main body 57.

The wings 24 preferably comprises means for attaching one face of the wing 24 to the wearer's undergarment or to the other wing 24. The wings 24 comprises at least one layer of adhesive 20. The attachment means may be pressure sensitive adhesive. If pressure sensitive adhesive is selected, it should be disposed on the face of the wing generally coextensive of the backsheet 16 so that when the wing 24 are wrapped around the crotch portion of the wearer's undergarment, the layer of adhesive will contact the outside of the wearer's undergarment. The sanitary napkin 10 for example comprises a generally rectangular patch of adhesive on each wing 24.

The sanitary napkin 10 comprises at least one layer of adhesive 20 to fasten the sanitary napkin 10 to an undergarment. In order to protect the layer of adhesive 20 prior to its use, the sanitary napkin 10 comprises a release paper 22 and/or release liner(s) 47 as illustrated in FIG. 1, to cover directly or indirectly said at least one layer of adhesive 20 each release paper 22 and/or release liner(s) 47 preferably comprising a layer or coating of peeling agent. Said at least one layer of adhesive 20 is arranged on one side of the backsheet 16, namely on the garment-facing side 19 of the backsheet 16, the backsheet 16 being arranged between the layer of adhesive 20 and absorbent core 12 as illustrated in FIG. 1. In other words, the at least one layer of adhesive 20 is arranged between the backsheet 16 and the release paper 22 and/or release liner(s) 47. According to the present disclosure, the release paper 22 can cover the layer of adhesive 20 arranged on both the main body 57 region and on the wings 24.

The at least one layer of adhesive 20 is arranged on the backsheet 16 in a discontinuous pattern of adhesive wherein said pattern of adhesive comprises a first area of adhesive 40 arranged in main body 57 region and a second area of adhesive 45 arranged in the wings 24 region. As illustrated in FIG. 2, the layer of adhesive 20 is arranged in a discontinuous pattern of adhesive comprising said first area of adhesive 40 extending in the front, central and rear portions F, C, R in the main body 57 region and a second area of adhesive 45 comprising a strip of adhesive material being arranged on each wing 24. The second area of adhesive 45 is arranged between the proximal and distal ends 25,27 of each wing 24. More specifically, the first area of adhesive 40 is arranged on the main body 57, i.e. on the backsheet at the main body 57 portion and the second area of adhesive 45 is arranged on the wings 24 which are outboard of the main body 57 with respect to the transversal direction T. The first area of adhesive 40 and/or the second area of adhesive 45, comprising preferably pressure sensitive adhesive can be applied continuously or discontinuously, in blocs or stripes, in dots or in swirls or any kind of common application, such as in triangles, stars, *etc.* As illustrated in FIG. 2, the first area of adhesive 40 extending in the front, central and rear portions F, C, R in the main body 57 is also arranged on the backsheet 16 in a discontinuous sub-pattern of adhesive wherein said sub-pattern of adhesive comprises a first sub-area of adhesive 40f arranged in the front portion F, a second sub-area of adhesive 40c arranged in the central portion C and a third sub-area of adhesive 40r arranged in the rear portion R. As illustrated in FIG. 2, the first, second and third sub-areas 40f,40c,40r of adhesive are not connected, or are isolated or separate from one another. In other words, the sanitary napkin 10 does not comprise any adhesive at the folding lines 29.

The present disclosure focus on the maintenance of the absorbent article 10 in a folded state by the release paper 22, hence the absorbent article 10 can comprise adhesive at the folding lines 29.

Figure 8 illustrates an absorbent article 10 selected from a pantyliner or an incontinence pad. A pantyliner or incontinence pad 10 comprises a topsheet 14, a backsheet 16, an absorbent core 12 arranged between said topsheet 14 and backsheet 16, and does not comprise any wings. In other words, the pantyliner or incontinence pad 10 does not present any section outboard of the longitudinal sides 32. As illustrated in FIG. 8, the pantyliner or incontinence pad 10 extends in longitudinal, transversal and vertical directions L,T,V, the pantyliner or incontinence pad 10 has a longitudinal centerline y-y, extending in the longitudinal direction L₁₀, a pair of transverse sides 30, meaning a pair of sides arranged at each longitudinal ends or longitudinal extremities, of the pantyliner or incontinence pad 10, and a pair of longitudinal sides 32 extending therebetween, meaning a pair of sides arranged along each transversal ends of the pantyliner or incontinence pad 10 and connecting the transverse sides 30 to one another. Similarly, the pair of transverse sides 30 connects the two longitudinal sides 32 to one another. In other words, the pantyliner or incontinence pad 10 comprises two ends, or extremities, at least partially extending in the transversal direction T, *i.e.* the transversal sides 30, and two ends at least partially extending in the longitudinal direction L₁₀, *i.e.* the longitudinal sides 32. As illustrated in FIG. 8, the transversal sides 30 are arcuate whereas the longitudinal sides 32 are linear. The pantyliner or incontinence pad 10 has a front edge 34 and a rear edge 36 positioned on opposite apexes of said transverse sides 30, meaning at each longitudinal extremities of the pantyliner or incontinence pad 10. The pantyliner or incontinence pad 10 is divided with respect to the longitudinal direction L, into three sections or portions, a front portion F, a rear portion R and a central portion C arranged between said front and rear portions F,R. The front, central and rear portions F,C,R are divided by the folding lines 29. The front, central and rear portions F,C,R can be equal or substantially equal in dimensions, e.g. 1/3 of the total length of the absorbent article 10, or of different dimensions, namely of same length or of different lengths, e.g. lengths of front and rear portions F,R are 30% of total length and length of central portion is 40% of total length of the absorbent article 10.

As illustrated in FIG. 8, the pantyliner or incontinence pad 10 comprises one region, a main body 57. The main body 57 is the region, or portion of the absorbent article 10 comprising the absorbent core 12 and the eventual ADL 18 and is oblong or oblong-shaped, meaning comprising a shape, e.g. a rectangular, that is longer than it is wide and whose corners are rounded. The main body 57 can be rectangular, or trapezoidal.

The pantyliner or incontinence pad 10 comprises at least one layer of adhesive 20 to fasten pantyliner or incontinence pad 10 to an undergarment. In order to protect the layer of adhesive 20 prior to its use, the pantyliner or incontinence pad 10 comprises a release paper 22 as described hereabove, to cover directly or indirectly said at least one layer of adhesive 20 the release paper 22 preferably comprising a layer or coating of peeling agent. Said at least one layer of adhesive 20 is arranged on one side of the backsheet 16, namely on the garment-facing side 19 of the backsheet 16, the backsheet 16 being arranged between the layer of adhesive 20 and absorbent core 12 as illustrated in FIG. 1. In other words, the at least one layer of adhesive 20 is arranged between the backsheet 16 and the release paper 22. According to the present disclosure, the release paper 22 covers the layer of adhesive 20 arranged on the main region 57.

The at least one layer of adhesive 20 is arranged on the backsheet 16 in a discontinuous pattern of adhesive wherein said pattern of adhesive comprises a first area of adhesive 40 arranged in main body 57 region. As illustrated in FIG. 8, the layer of adhesive 20 is arranged in a discontinuous pattern of adhesive comprising said first area of adhesive 40 extending in the front, central and rear portions F, C, R in the main body 57 region. More specifically, the first area of adhesive 40 is arranged on the main body 57, i.e. on the backsheet at the main body 57 portion. The first area of adhesive 40 comprises preferably pressure sensitive adhesive and can be applied continuously or discontinuously, in blocs or stripes, in dots or in swirls or any kind of common application, such as in triangles, stars, *etc.* As illustrated in FIG. 8, the first area of adhesive 40 extending in the front, central and rear portions F, C, R in the main body 57 is also arranged on the backsheet 16 in a discontinuous sub-pattern of adhesive wherein said sub-pattern of adhesive comprises a first sub-area of adhesive 40f arranged in the front portion F, a second sub-area of adhesive 40c arranged in the central portion C and a third sub-area of adhesive 40r arranged in the rear portion R. As illustrated in FIG. 8, the first, second and third sub-areas 40f,40c,40r of adhesive are not connected, or are isolated or separate from one another. In others words, the pantyliner or incontinence pad 10 does not comprise any adhesive at the folding lines 29.

According to an embodiment, both the first area of adhesive 40 comprise, preferably consists essentially of, pressure sensitive adhesive, such embodiment simplifies the process as only one material can be used for the adhesive.

The present disclosure is not limited to one specific embodiment and some embodiment can be combined. Sanitary napkins and pantyliners or incontinence pads 10 differ in construction with respects to the wings, however some features can be transposed from one embodiment to the other. For example, the pattern of the first area of adhesive 40 is not limited to one type of absorbent article 10 and can be used in sanitary napkins and pantyliners and incontinence pads.

Absorbent articles 10 are sold in a folded state, either in a three-time fold, meaning comprising two folding lines or that the sanitary napkin has been folded twice, or in a two-time fold, meaning comprising one folding line or that the sanitary napkin has been folded once. It is possible to also have absorbent articles 10 sold in a four-time fold comprising three folding lines. Such folding enables to fit more articles in a rectangular carboard box or plastic bag.

An absorbent article 10 comprising a three-time fold is such that the absorbent article 10 is folded at two folding lines 29 delimiting, or separating, the front, central and rear portions F,C, R. Both front and rear portions F,R are folded inwardly, sequentially or simultaneously, towards the central portion C, each around a folding line 29 to obtain an absorbent article 10 in a folded configuration. In order to maintain this article in a folded configuration, the release paper 22 is then folded around the proximal end 25 onto the folded front and rear portions F,R.

As illustrated in FIG. 3, the absorbent article 10 is arranged in a final or fully folded configuration. The folded absorbent article 10 extends in longitudinal, transversal and vertical directions (L,T,V), the front and rear portions (F,R) are folded onto the central portion (C). As illustrated in FIG. 3, the front portion F is folded directly onto the central portion and the rear portion R is folded directly onto the front portion F and thus indirectly onto the central portion C. The present disclosure is not limited to this arrangement and the rear portion R can be folded directly onto the central portion C and the front portion F can be folded directly onto the rear portion R and indirectly onto the central portion C.

When present the wings can be folded onto the central portion C or onto the front portion F or onto the rear portions R, meaning the wings, when present can be folded directly onto the central portion C or the wings can be folded directly onto the front portion F and indirectly onto the central portion C or the wings can be folded directly onto the rear portion R and indirectly onto the front and central portions F,C. Hence, with respect to the vertical direction V, the sanitary napkin 10 in a final or fully folded configuration, is arranged with the wings and front portion F being arranged between the central portion C and the rear portion R or the front and rear portions F,R being arranged between the central portion C and the wings, with the release paper 22 surrounding or enveloping the front or rear portion F,R or wings and central portion C.

As illustrated in FIG. 3, the absorbent article 10 is folded in three, meaning comprising two folding lines 29, and is maintained in a folded configuration, or folded state, or folded conformation, by the release paper 22. In order words, the release paper 22 acts as clamping mean maintaining the absorbent article 10 in a folded state. As shown in FIG. 3, the release paper 22 is overlaying the wings when present, front and rear portions F,R and the central portion C is underlaying the wings when present and front and rear portions F,R and the release paper 22 is underlaying the central portion C as well with respect to the vertical direction V.

As illustrated in FIG. 3 and 9, the assembly 1 comprising the release paper 22 and the absorbent article 10 such as a sanitary napkin, pantyliner or incontinence pad, extends in the longitudinal, transversal and vertical directions (L,T,V). In other words, the release paper 22 and the absorbent article 10 extend in a three-dimensional space defined by the longitudinal, transversal and vertical directions (L,T,V). The absorbent article 10 extends in these same directions such that the length of the absorbent article 10, corresponding to the greatest dimension with respect to the width or height, is in the same longitudinal direction L as defined by the assembly 1, or in other words the longitudinal direction L₁₀ of the absorbent article 10 is parallel to the longitudinal direction of the assembly 1. The release paper 22, according to the present disclosure, extends in these same directions, meaning in the same three-dimensional space such that the length of the release paper 22, corresponding to the greatest dimension with respect to the width or height, is in the transversal direction T as defined by the assembly 1, or in other words the longitudinal direction L₂₂ of the release paper 22 is orthogonal to the longitudinal direction of the assembly 1. In other words, the longitudinal direction L₂₂ of the release paper 22 is orthogonal to the longitudinal direction L₁₀ of the absorbent article 10, this is especially apparent when the assembly 1 is in an unfolded configuration as illustrated in FIG. 9.

As illustrated in FIG. 3, when the assembly 1 is in a folded of final configuration, a gap 28 is arranged or present between the two longitudinal extremities 72 of the release paper 22. In other words, the two longitudinal extremities 72 of the release paper 22 are separated by a gap 28. Said gap 28 is between 0.1 cm and 10 cm, preferably between 0.1 and 5 cm, more preferably between 0.1 cm and 2.5 cm.

In other to maintain the absorbent article 10 in a folded state, the absorbent article 10 can comprise additional means to ensure this clamping means.

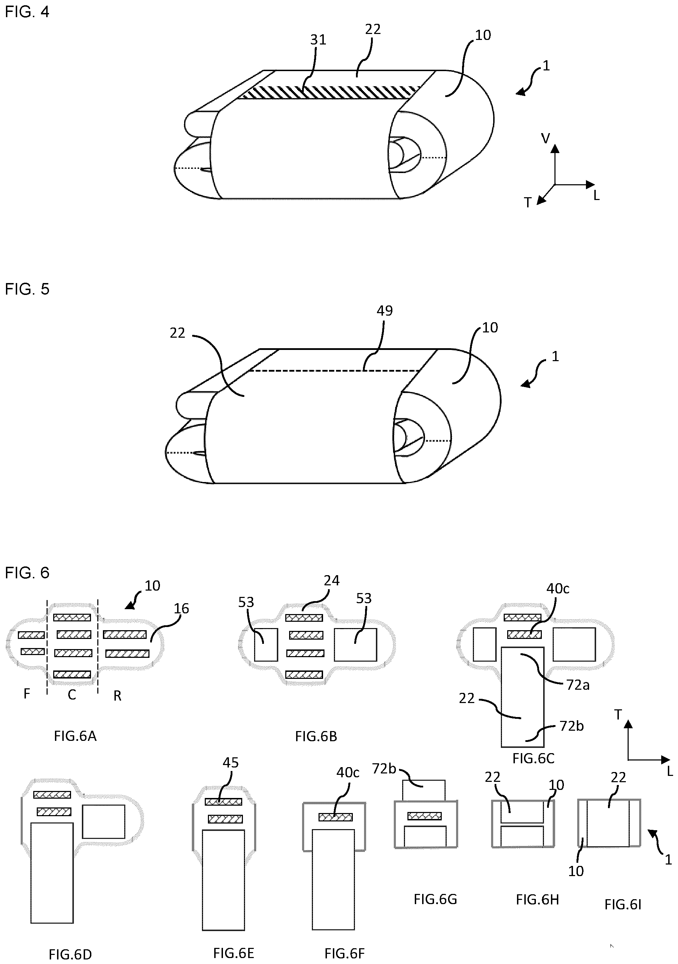

For example, as illustrated in FIG.4, in a folded or final configuration, the release paper 22 can comprise an overlapping region 31, where the two longitudinal extremities 72a,72b of the release paper 22 are overlapping with adhesive material being arranged in said overlapping region 31, meaning on the shortest distance between the two longitudinal extremities 72 of the release paper 22. In other words, the words, one longitudinal extremity 72a, 72b overlays the other, or opposite, longitudinal extremity 72a, 72b with adhesive material being arranged between the two longitudinal extremities 72a,72b. In other words, with respect to the vertical direction in the overlapping region 31, the release paper 22 comprises one longitudinal extremity, adhesive material and the other longitudinal extremity. The release paper 22 can also be a one-piece ring, or an annular monolithic paper forming continuity of mater, surrounding the absorbent article 10, said release paper comprising pre-cuts 49 or perforations 49 or a weakened division line 49 as shown in FIG. 5.

According to the present disclosure, the absorbent article is arranged in a folded configuration such that absorbent article is maintained directly or indirectly in said folded configuration by the release paper. By arranging the absorbent article, or the absorbent article, in a way that the release paper can maintain said absorbent article in a folded configuration thereby removing the need altogether for wrapper, the environmental impact of the packaging is lowered since less material is used.

According to an embodiment, given that the objective is to reduce the environmental impact of the assembly 1 comprising an absorbent article 10 and a release paper 22, the release paper 22 can be partially coated, such that the coating of peeling agent is equal to or slightly greater than the first area of adhesive 40. According to another embodiment, the release paper 22 and/or the release liner 47 is partially coated such that the coating of peeling agent is equal or slightly greater than the first area of adhesive 40 and/or second area of adhesive 45 respectively. As shown in FIG. 2, when the layer of adhesive 20, namely the first and/or second area of adhesive 40,45, is applied in a discontinuous pattern, the release paper 22 will therefore cover zones of the backsheet 16 that does not comprise any adhesive. It can be beneficial to have a release paper 22 that is not fully coated with peeling agent thereby saving on raw material, namely peeling agent such as silicone.

Indeed, the release paper 22 and/or release liner 47 of the present disclosure has peelable properties with an area of a surface of the release paper 22 and/or release liner 47 being subjected to peeling treatment(s) as described hereabove. For example, the area of the surface of the release paper 22 and/or release liner 47 with which the layer of adhesive 20 of the absorbent article 10 is in contact, and eventually its vicinity, can be subjected to said peeling treatment(s) namely the coating of a layer of peeling agent such as silicone. The area, or surface area, of peeling agent can be equal to or slightly greater than the area, or surface area, defined by the layer of adhesive 20. As seen above, the layer of adhesive 20 is applied discontinuously on a portion of the backsheet 16 of the absorbent article 10. The area of peeling agent can also be arranged, or applied or coated, discontinuously on a surface of the release paper 22 and/or release liner 47. For example, the area, or surface area, of peeling agent 32 is greater than 30%, preferably comprised between 40% and 95%, more preferably between 50% and 90% of the total area of a surface of the release paper 22 and/or release liner 47.

According to an embodiment, the ratio between the total surface area of peeling agent arranged on the release paper 22 and/or release liner 47 and the total surface area of adhesive arranged on the backsheet 16 is comprised between 1 and 1.5, preferably between 1 and 1.2, more preferably between 1 and 1.1, even more preferably between 1 and 1.05, even more preferably said ratio is equal to 1. For example, the ratio between the total surface area of peeling agent arranged on the release paper 22 and the total surface area of adhesive arranged on the main body 57 region is 1.4 and/or the ratio between the total surface area of peeling agent arranged on each release liner 47 and the total surface area of adhesive 45 arranged on each wings 47 is 1.2. According to these embodiments, the area of peeling agent and/or adhesive arranged on the backsheet 16 is preferably arranged discontinuously on a surface of the release paper 22 and/or release liner 47.

According to an embodiment, the ratio between the total surface area of the release paper 22 and eventually release liner 47 and the total surface area of adhesive arranged on the backsheet 16 is comprised between 1 and 1.5, preferably between 1 and 1.2, more preferably between 1 and 1.1, even more preferably between 1 and 1.05, even more preferably said ratio is equal to 1. According to these embodiments, the area of adhesive arranged on the backsheet 16 can be arranged continuously, meaning forming a continuity of matter, or discontinuously.

The present disclosure also pertains to a method for folding an absorbent article 10 as described herein, the method comprises the following steps:
- providing an absorbent article as described herein, preferably laid flat on a surface and extending in a longitudinal, transversal and vertical direction;
- applying a release tape 53 onto the front and/or rear portion (F,R) to cover the first and/or third sub-area 40f,40r of adhesive;
- applying the release paper 22 in a direction orthogonal to the longitudinal direction of the absorbent article 10 with one longitudinal extremity 72, meaning a first longitudinal extremity 72a, of the release paper 22 being applied onto a first portion of the second sub-area 40c of adhesive and optionally on the second area of adhesive 45 present on one of the wings 24 when present; in other words, one longitudinal end of the release paper 22 covers a first part of the sub-area 40c of adhesive present on the central region C of the absorbent article 10;
- folding the front portion F onto the central portion C and folding the rear portion onto the front portion or folding the rear portion R onto the central portion C and folding the front portion F onto the rear portion R; the front portion F being folded around a folding line 29; namely the folding line 29 dividing the front and central portions F,C and the rear portion R being folded around a folding line 29 namely the folding line 29 dividing the rear and central portions R,C;
- optionally folding the wings 24 onto the folded front and rear portions (F,R); in other words, when present folding the wings 24 onto the folded front and rear portions (F,R);
- wrapping or folding the release paper 22 around the folded absorbent article 10 onto the folded front and rear portion or onto the folded wings when present; and
- securing or fastening the release paper 22 to the other or second portion of the second sub-area 40c of adhesive, meaning securing or applying the second longitudinal extremity 72b of the release paper 22 to the second portion of the second sub-area 40c of adhesive

These steps are preferably sequential although some steps can be simultaneous. These steps are preferably in this sequence.

The release tape 53 is preferably siliconized paper with a paper layer is coated with a layer of peeling agent selected from silicon resin series, fluororesin series, octadecilisocyanate series. In other words, the release tape 53 is similar in structure to the release paper 22 and/or release liner 47.

The sequence of FIG.6 (FIG. 6A to 6I) exemplifies one embodiment of the method according to a bottom-view, meaning when looking directly at the backsheet 16. FIG. 6A to 6I depicts the different steps in said method, the absorbent article 10, such as a pantiliner or incontinence pad or a sanitary napkin 10 as described hereabove. As shown in FIG. 6A a sanitary napkin 10 is provided and arranged flat in an unfolded configuration. The absorbent article 10, extends in a three dimensional space defined by the longitudinal L, transversal T and vertical (not shown) axis as illustrated in FIG. 6.

As shown in FIG. 6B, a release tape 53 is applied onto each front and rear portions (F,R) to cover the first and third sub-areas 40f,40r of adhesive in the first area of adhesive 40 in the main portion 57. In other words, a release tape 53 is placed to cover the patch of adhesive present in both the front and rear portions F,R.

As illustrated in FIG. 6C, a release paper 22 is then applied onto a portion, e.g. a half, of the second sub-area 40c of adhesive and optionally onto one wing 24, namely onto the area of adhesive 45 present on one wing 24. In the embodiment as illustrated in FIG. 6, the second sub-area 40c of adhesive, present in the central portion C, comprises two parallel stripes of adhesive arranged at a distance with respect to the transversal direction T. The release paper 22 can be arranged so as to cover and thereby be attached to one of said stripes. As show in FIG. 6C, the release paper 22 is arranged such that the longitudinal direction of the release paper 22 is orthogonal to the longitudinal direction of the absorbent article 10 or in other words, the release paper 22 extends in length in the transversal direction T of the absorbent article 10. To further specify, a first longitudinal extremity 72a of the release paper 22 is substantially applied onto a first portion of the second sub-area 40c of adhesive, with the second, or opposite, longitudinal extremity 72b of the release paper 22 extending outboard of the absorbent article 10. The release paper 22 covers only a portion of the second sub-area 40c of adhesive and not its entirety.

As shown in FIG. 6D, the front portion F is folded onto the central portion C and the front portion F is then folded onto the folded rear portion R as depicted in FIG. 6E. The release tapes 53 enable that the first and third sub-areas of adhesive 40f,40r do not stick onto the topsheet 14 and onto the backsheet 16. Specifically, the release tape 53 in the front portion F enables that the first sub-area 40f of adhesive does not stick onto the topsheet 14 of the rear portion R and the release tape 53 in the rear portion R enables that the third sub-area 40r of adhesive does not stick onto the wings 24 when present. The present disclosure is not limited to this embodiment, for example the rear portion R can be folded onto the central portion C and the front portion F can be folded onto the folded rear portion R. For example, when the absorbent article 10 is a pantyliner or an incontinence pad and no wings are present, only one release tape 53 can be applied on the absorbent article 10, either onto the front portion F or onto the rear portion R, namely on whichever portion is folded first onto the central portion C. Of course, even if the absorbent article 10 is wingless such as a pantyliner, a release tape 53 can be applied on both the front and rear portions F,R.

The front and/or rear portions F,R are folded inwardly, meaning towards the tospheet 14 or towards the side of the absorbent article 10 where the tophseet is arranged. In other words, the front and/or rear portions F,R are folded towards the body-facing side 17.

As shown in FIG. 6F, when present, the method comprises the step of folding both wings 24, either sequentially or simultaneously, onto the folded front and rear portions (F,R), here directly over the rear portion (R), more specifically, the wings 24 are folded onto the portion, front or rear F,R, which was folded last. One of the wings 24 can drag a portion of the release paper 22 to be folded at least partially.

As illustrated in FIG. 6G, the release paper 22 is then folded around the absorbent article 10 thereby wrapping the release paper 22 at least partially around the folded absorbent article 10 for example onto the folded wings 24 when present or onto the rear portion R when folded onto the front portion, for example the release paper 22 can cover the second area of adhesive 45 or the third sub-area 40r of adhesive or the release tape 53 covering the third sub-area 40r. In other words, the release paper 22 can be folded by a 180°-fold. The release paper 22 is folded and extends beyond the absorbent article 10 with respect to the transversal direction as shown in FIG. 6G.

As illustrated in FIG. 6H, the release paper 22, namely the second longitudinal extremity 72b of the release paper 22 that was folded in the previous step, is then folded onto the other, or second, portion of the second sub-area 40c of adhesive that was not covered by the first longitudinal 72a extremity of the release paper 22. FIG. 6H and FIG. 6I are illustrations of the absorbent article 10 and release paper 22 in a final folded configuration. The assembly 1 comprising the absorbent article 10 and release paper 22 is maintained in a folded configuration by the release paper 22 since the release paper 22 is folded around the absorbent article 10 and both longitudinal extremities of the release paper 22 are joined to both patch of adhesive 40c in the central region C, meaning to the second-sub area 40c of adhesive. FIG. 6H correspond to the absorbent article from a bottom view, same view as in FIG. 6A, whereas FIG. 6I corresponds to the to the absorbent article from a top view.

In addition, the absorbent article 10 is protected by the release paper 22. In a folded configuration, the total surface area of the folded release paper 22 covers preferably between 50% and 100 % of the total surface area of the folded absorbent article 10, more preferably, between 60% and 99%, even more preferably, between 70% and 98%. The total surface area of the release paper 22 and the absorbent article 10 is as shown in FIG. 6H and 6I. For sake of clarity, in a final folded configuration, the total apparent surface area of the release paper 22 covers preferably between 50% and 100 % of the total apparent surface area of the absorbent article 10, more preferably, between 60% and 99%, even more preferably, between 70% and 98%.

The present disclosure is not limited to this method embodiment alone. There may be other ways to ensure that the release paper 22 maintains the absorbent article 10 folded, or in a folded configuration.

For example, for embodiment where the release paper 22 comprises an overlapping region 31, the first longitudinal extremity 72a can be applied on the entirety of the second sub-area 40c of adhesive since the second longitudinal extremity 72b will be fastened to the first longitudinal extremity 72a and maintain the absorbent article folded. Hence, the present disclosure also pertains to a method for folding an absorbent article 10 as described herein, the method comprises the following steps:
- providing an absorbent article as described herein, preferably laid flat on a surface and extending in a longitudinal, transversal and vertical direction;
- applying a release tape 53 onto the front and/or rear portion (F,R) to cover the first and/or third sub-area 40f,40r of adhesive;
- applying the release paper 22 in a direction orthogonal to the longitudinal direction of the absorbent article 10 with a first longitudinal extremity 72 of the release paper 22 being applied to cover at least partially, preferably entirely, the second sub-area 40c of adhesive and optionally on the second area of adhesive 45 present on one of the wings 24 when present; in other words, one longitudinal end of the release paper 22 covers at least a first part, preferably the entirety, of the second sub-area 40c of adhesive present on the central region C of the absorbent article 10;
- folding the front portion F onto the central portion C and folding the rear portion onto the front portion or folding the rear portion R onto the central portion C and folding the front portion F onto the rear portion R; the front portion F being folded around a folding line 29; namely the folding line 29 dividing the front and central portions F,C and the rear portion R being folded around a folding line 29 namely the folding line 29 dividing the rear and central portions R,C;
- optionally folding the wings 24 onto the folded front and rear portions (F,R); in other words, when present folding the wings 24 onto the folded front and rear portions (F,R);
- wrapping or folding the release paper 22 around the folded absorbent article 10 onto the folded front and rear portion or onto the folded wings when present; and
- applying adhesive onto the first longitudinal extremity 72a of the release paper in an overlapping region31;
- securing the second longitudinal extremity 72b to the first longitudinal extremity 72a in said overlapping region 31.

Of course the present disclosure is not limited to these embodiments and the above methods can be combined, for example even with a release paper 22 with an overlapping region 31, the first longitudinal extremity 72a can still be applied on a portion of the second sub-area 40c and not the entirety.

The sequence of FIG.7 (FIG. 7A to 7G) exemplifies another embodiment of the method according to a top-view, meaning when looking directly at the topsheet 14. FIG. 7A to 7G depicts the different steps in said method, the absorbent article 10, such as a pantiliner or incontinence pad or a sanitary napkin 10 as described hereabove. As shown in FIG. 7A a pantiliner or an incontinence pad 10 is provided and arranged flat in an unfolded configuration. The absorbent article 10, extends in a three dimensional space defined by the longitudinal L, transversal T and vertical (not shown) axis as illustrated in FIG. 7.

The method comprises the following steps:
- providing an absorbent article 10 as described herein, preferably laid flat on a surface and extending in a longitudinal, transversal and vertical direction (FIG. 7A);
- when present folding the wings onto the central portion C and applying a release liner 47 to keep the wings folded onto the central portion;
- folding the front portion F onto the central portion C (FIG. 7A to 7B), the front portion F being folded around a folding line 29; namely the folding line 29 dividing the front and central portions F,C;
- applying a release paper 22 in a direction orthogonal to the longitudinal direction of the absorbent article 10 with one, or a first, longitudinal extremity 72 of the release paper 22 being applied onto the first sub-area 40f of adhesive (FIG. 7C); as shown in FIG. 7C, the release paper 22 covers at least partially, preferably entirely, the first sub-area 40f of adhesive;
- folding the rear portion R directly onto the release paper 22 and indirectly onto the front portion F (Fig. 7C to 7D), the rear portion R being folded around another folding line 29, namely the folding line 29 dividing the rear and central portions R,C;
- wrapping or folding the release paper 22 around the folded absorbent article 10 onto the second sub-area 40c of adhesive (Fig. 7D to 7E) ; and
- securing or fastening the release paper 22 to the third sub-area 40r of adhesive, or applying the free end of the release paper 22, or the second longitudinal end 72b, onto the third sub-area 40r of adhesive arranged on the rear portion R (Fig. 7E to 7F) as shown in FIG. 7F, the release paper 22 covers at least partially, preferably entirely, the third sub-area 40r of adhesive.

These steps are preferably sequential although some steps can be simultaneous. These steps are preferably in this sequence.

The front and/or rear portions F,R are folded inwardly, meaning towards the tospheet 14 or towards the side of the absorbent article 10 where the tophseet is arranged. In other words, the front and/or rear portions F,R are folded towards the body-facing side 17. The present disclosure is not limited to this illustrated example, for example, the first step can comprise the folding the rear portion (R) onto the central portion (C) and then the folding of the front portion onto the rear portion.

As illustrated in FIG. 7E, the release paper 22 is folded around the absorbent article 10 thereby wrapping the release paper 22 at least partially around the folded absorbent article 10 for example onto the rear portion C, for example the release paper 22 can cover the second sub-area 40c of adhesive arranged on the central portion C. In other words, the release paper 22 can be folded by a 180°-fold when compared to the previous step as shown in FIG. 7D. The release paper 22 is folded and extends beyond the absorbent article 10 with respect to the transversal direction as shown in FIG. 7E.

As illustrated in FIG.7F, the release paper 22, namely the second longitudinal extremity of the release paper 22 that was folded in the previous step, is then folded onto the third sub-area 40r of adhesive arranged on the rear portion R. FIG.7F and 7G are an illustration of the absorbent article 10 and release paper 22 in a final folded configuration according to a top and bottom view respectively.

The assembly 1 comprising the absorbent article 10 and release paper 22 is maintained in a folded configuration by the release paper 22 since the release paper is folded around the absorbent article 10 and both longitudinal extremities 72 of the release paper 22 are joined to both patch of adhesive in the front and rear region F,R, meaning the first and third sub-areas of adhesive 40f,40r. FIG. 7F corresponds to the absorbent article from a top view, same view as in FIG. 7A, whereas FIG. 7G corresponds to the to the absorbent article from a bottom view.

When seeing the absorbent article 10 as illustrated in FIG. 6H, 6I, 7F or 7G, it is apparent that such absorbent article 10 corresponds to a more environmentally friendly absorbent article. Such absorbent article 10 is free of wrapping plastic film or plastic pouch and the topsheet 14 and absorbent core 12, meaning the portion of the absorbent article that is in contact with the wearer's body is well protected by the backsheet 16 and the release paper 22. The topsheet 14 is also well protected since the release paper 22 maintain the absorbent article 10 in a folded configuration.

According to the present disclosure the method illustrated in FIG. 6 (6A to 6I) can be applied to fold a wingless article 10, and the method illustrated in FIG. 7 (6A to 6I) can be applied to fold a absorbent article 10 with wings.

Hence the release paper 22 according to the present disclosure acts as a clamping mean, or a holding mean, for the absorbent article 10 and keeps it in a folded configuration while saving on material and thus being more environmentally friendly. Additionally, by not using a conventional pouch, there is no need to add a closing tape with the present assembly 1 again saving on material.

According to a further embodiment, the release paper 22, release liner 47, release tape(s) 53 and/or the backsheet 16 can also comprise a graphic element printed upon its surface. The graphic elements on the release paper 22, release tape(s) 53, release liner 47 and/or backsheet 16, can be matching or congruent. By congruent it is meant capable of being placed over another figure and exactly matching, for example if a circle is printed on the release paper 22, release tape(s) 53, the backsheet 16 or release liner 47 comprises two arcuate lines matching the partial periphery of said circle. The graphic element printed on the release paper 22 and/or backsheet 16 can be of the same nature as the graphic element printed on the release liner 47 or release tape(s) 53, or of different nature. For example the release paper 22 and/or backsheet 16 can comprise a pattern printed upon its outer surface whereas the wrapper comprises one or more words printed upon its outer surface or the example the release paper 22 and/or backsheet 16 and the wrapper can all comprise a drawing printed upon its surface. According to a further embodiment, the release paper 22, the backsheet 16, release tape(s) 53 and the release liner 47 each comprises a graphic element.

As described above, the release paper 22, release tape(s) 53 and/or release liner 47 are preferably siliconized paper with a paper layer is coated with a layer of peeling agent selected from silicon resin series, fluororesin series, octadecilisocyanate series. According to an embodiment, the peeling layer comprises a basis weight comprised between 0,3 gsm and 3 gsm, preferably between 0,5 gsm and 1 gsm and the paper layer comprises a basis weight comprised between 10 gsm and 40 gsm. This release paper 22, release tape(s) 53 and/or release line 47 are preferably recyclable.

The layer of adhesive 20 comprises the first area of adhesive 40 and the second area of adhesive 45 preferably comprises pressure sensitive adhesive as such adhesive is easy to attach and remove from the garment. Specially, the first, second and third sub-areas 40f, 40c,40r of adhesive comprise pressure sensitive adhesive. Example of pressure sensitive adhesive comprises and are not limited to styrene block copolymers (SBC), solvent-acrylic, emulsion-acrylic, silicone or solvent-rubber based material. The layer of adhesive 20 can comprise other types of adhesive such as non-petroleum derived material as to lower the environmental impact of the absorbent article. The adhesive are preferably selected from and not limited to polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL), a polymer material such as a polyethylene material, e.g. as a low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid. The adhesive and/or heat-sealable material preferably comes from a sustainable source, or bio-based or bioplastic, and is biodegradable and/or compostable.

As seen above, the release paper 22 itself can reduce the environmental impact of the assembly by using less material, i.e. less silicone. According to another aspect of the present disclosure, the present disclosure also pertains to an assembly 1 comprising a release paper 22 and an absorbent article 10, the absorbent article 10 comprising a topsheet 14, a backsheet 16, an absorbent core 12 arranged between said topsheet 14 and backsheet 16, and a layer of adhesive 20 being arranged on a side of the backsheet 16 opposite to the absorbent core 12, the release paper 22 being arranged on a side of the layer of adhesive 20 opposite to the backsheet 16 wherein the release paper 22 is partially coated by a coating of peeling agent, such that the coating of peeling agent is equal to or slightly greater than the first area of adhesive 40. The following is with respect to this other aspect of the present disclosure.

According to an embodiment, the release paper 22 and/or the release liner 47 is partially coated such that the coating of peeling agent is equal or slightly greater than the first area of adhesive 40 and/or second area of adhesive 45 respectively. When the layer of adhesive 20, namely the first and/or second area of adhesive 40,45, is applied in a discontinuous pattern, the release paper 22 will therefore cover zones of the backsheet 16 that does not comprise any adhesive. It can be beneficial to have a release paper 22 that is not fully coated with peeling agent thereby saving on raw material, namely peeling agent such as silicone. the release paper 22 and/or release liner 47 of the present disclosure has peelable properties with an area of a surface of the release paper 22 and/or release liner 47 being subjected to peeling treatment(s) as described hereabove. For example, the area of the surface of the release paper 22 and/or release liner 47 with which the layer of adhesive 20 of the absorbent article 10 is in contact, and eventually its vicinity, can be subjected to said peeling treatment(s) namely the coating of a layer of peeling agent such as silicone. For example, the layer of adhesive 20 is applied continuously between the front, central and/or rear portions F,C,R in two or more stripes extending parallel and elongated in the longitudinal direction, the release paper 22 can be as short and/or as thin than these stripes of adhesive material or the release paper 22 can be wider and/or longer than these stripes of adhesive material but comprise a peeling coating that is as short and/or as thin than these stripes of adhesive material, or in other words, the dimensions, namely the length and width, of the release paper and/or coating of peeling agent is equal to the dimensions, namely the length and width, of the stripes of adhesive material. Similarly, the dimensions, namely the length and width, of the release liner and/or coating of peeling agent is equal to the dimensions, namely the length and width, of the stripes of adhesive material on the wings. In other words, the release paper and the adhesive on the backsheet can be congruent, meaning are of same size and shape and are placed one over the other and exactly matching.

The area of peeling agent can be equal to or slightly greater than the area defined by the layer of adhesive 20. As seen above, the layer of adhesive 20 is applied discontinuously on a portion of the backsheet 16 of the absorbent article 10. The area of peeling agent can also be arranged, or applied or coated, discontinuously on a surface of the release paper 22 and/or release liner 47. In other words, the adhesive can be applied on the backsheet 16 in a discontinuous pattern, same as the peeling agent which can be applied in a discontinuous pattern on the release paper 22. For example, the area of peeling agent 32 is greater than 30%, preferably comprised between 40% and 95%, more preferably between 50% and 90% of the total area of a surface of the release paper 22 and/or release liner 47.

According to an embodiment, the ratio between the total surface area of peeling agent arranged on the release paper 22 and/or release liner 47 and the total surface area of adhesive arranged on the backsheet 16 is comprised between 1 and 1.5, preferably between 1 and 1.2, more preferably between 1 and 1.1, even more preferably between 1 and 1.05, even more preferably said ratio is equal to 1. For example, the ratio between the total surface area of peeling agent arranged on the release paper 22 and the total surface area of adhesive arranged on the main body 57 region is 1.4 and/or the ratio between the total surface area of peeling agent arranged on each release liner 47 and the total surface area of adhesive 45 arranged on each wings 47 is 1.2. According to these embodiments, the area, or surface area, of peeling agent is preferably arranged discontinuously, meaning the peeling agent is arranged in a discontinuous pattern, on a surface of the release paper 22 and/or release liner 47, similarly, the area of adhesive is preferably arranged discontinuously, meaning the adhesive is arranged in a discontinuous pattern, on a surface of the backsheet 16. In other words, the peeling agent coated on the release paper and the adhesive on the backsheet are congruent, meaning are of same size and shape and are placed one over the other and exactly matching.

According to an embodiment, the ratio between the total surface area of the release paper 22 and eventually release liner 47 and the total surface area of adhesive arranged on the backsheet 16 is comprised between 1 and 1.5, preferably between 1 and 1.2, more preferably between 1 and 1.1, even more preferably between 1 and 1.05, even more preferably said ratio is equal to 1. According to these embodiments, the area of adhesive arranged on the backsheet 16 can be arranged continuously, meaning forming a continuity of matter, or discontinuously. In other words, the adhesive can be applied on the backsheet 16 in a continuous pattern. For example, the layer of adhesive 20 is applied continuously between the front, central and/or rear portions F,C,R in two or more stripes extending parallel and elongated in the longitudinal direction, the release paper 22 can be as short and/or as thin than these stripes of adhesive material or the release paper 22 can be wider and/or longer than these stripes of adhesive material but comprise a peeling coating that is as short and/or as thin than these stripes of adhesive material, or in other words, the dimensions, namely the length and width, of the release paper and/or coating of peeling agent is equal to the dimensions, namely the length and width, of the stripes of adhesive material. Similarly, the dimensions, namely the length and width, of the release liner and/or coating of peeling agent is equal to the dimensions, namely the length and width, of the stripes of adhesive material on the wings.

It is possible to combine the features of the different embodiments mentioned hereabove. For example a release line 47 can comprise a graphic element congruent or matching the graphic element of the release paper 22 and pre-cuts 49 i.e. a weakened division line 49. For example, the present disclosure pertains to an embodiment where an assembly 1 comprising a release paper 22 and an absorbent article (10), the absorbent article 10 as described hereabove, meaning comprising a topsheet 14, a backsheet 16, an absorbent core 12 arranged between said topsheet 14 and backsheet 16, and a layer of adhesive 20 being arranged on a side of the backsheet 16 opposite to the absorbent core 12, the release paper 22 being arranged on a side of the layer of adhesive 20 opposite to the backsheet 16, the release paper 22 and the absorbent article 10 extending in respective longitudinal, transversal and vertical directions (L,T,V), the absorbent article 10 is maintained in a folded configuration by the release paper 22 and in that the longitudinal direction of the release paper 22 is orthogonal to the longitudinal direction of the absorbent article 10 and the ratio between the total surface area of peeling agent arranged on the release paper 22 and/or release liner 47 and the total surface area of adhesive arranged on the backsheet 16 is comprised between 1 and 1.5. It is supposed that the present invention is not restricted to any form of realization described previously and that some modifications can be added to the presented example of fabrication without reappraisal of the appended claims.

## Claims

1. Assembly (1) comprising a release paper (22) and an absorbent article (10), the absorbent article (10) comprising :
- a topsheet (14),
- a backsheet (16),
- an absorbent core (12) arranged between said topsheet (14) and backsheet (16), and
- a layer of adhesive (20) being arranged on a side of the backsheet (16) opposite to the absorbent core (12),
the release paper (22) being arranged on a side of the layer of adhesive (20) opposite to the backsheet (16),
the release paper (22) and the absorbent article (10) extending in respective longitudinal, transversal and vertical directions (L,T,V),
**characterized in that** the absorbent article (10) is maintained in a folded configuration by the release paper (22) and **in that** the longitudinal direction of the release paper (22) is orthogonal to the longitudinal direction of the absorbent article (10).

2. Assembly (1) according to claim 1, wherein the absorbent article (10) extends in longitudinal, transversal and vertical directions (L,T,V), and comprises in an unfolded configuration with respect to the longitudinal direction, a front portion (F), a rear portion (R) and a central portion (C) arranged between said front and rear portions (F,R), when maintained in said folded configuration, the front and rear portions (F,R) are folded onto the central portion (C).

3. Assembly (1) according to any of the preceding claims, wherein the absorbent article (10) comprises wings (24), wherein each wing (24) comprises an area of adhesive (45) arranged on the side of the backsheet (16) opposite to the absorbent core (12).

4. Assembly (1) according to claim 2 or claims 2 and 3, wherein a layer of adhesive (20) comprises a first area of adhesive (40), said first area of adhesive being applied in a discontinuous pattern with a first sub-area of adhesive (40f) arranged in the front portion (F), a second sub-area of adhesive (40c) arranged in the central portion (C) and a third sub-area of adhesive (40r) arranged in the rear portion (R), said first, second and third sub-area of adhesive (40f, 40c, 40r) being distinct from one another.

5. Assembly (1) according to any of the preceding claims, the release paper (22) comprises an overlapping region (31) where the two longitudinal extremities (72a,72b) of the release paper (22) are overlapping with adhesive material being arranged in said overlapping region (31) between said two longitudinal extremities (72a,72b) of the release paper (22).

6. Assembly (1) according to any of the claims 1 to 4, wherein a gap (28) is arranged between the two longitudinal extremities (72a,72b) of the release paper (22).

7. Assembly (1) according to any of the preceding claims, wherein in a folded configuration the surface area of the folded release paper (22) covers between 50 and 99 % of the surface area of the folded absorbent article (10).

8. Assembly (1) according to any of the preceding claims, wherein the release paper (22) is partially coated by a peeling agent, such that the surface area of peeling agent is comprised between 40% and 95%, preferably between 50% and 90% of the total area of a surface of the release paper (22).

9. Assembly (1) according to claim 8, wherein the ratio between the total surface area of peeling agent arranged on the release paper (22) and the total surface area of adhesive arranged on the backsheet (16) is comprised between 1 and 1.5.

10. Method for manufacturing an assembly (1) comprising a release paper (22) and an absorbent article (10) according to the claims 2 to 9, wherein said method comprises the following steps :
- providing said absorbent article (10) and said release paper (22);
- applying a release tape (53) onto the front and/or rear portion (F,R) to cover the first and/or third sub-area (40f,40r) of adhesive;
- applying the release paper (22) in a direction orthogonal to the longitudinal direction of the absorbent article (10) with a first longitudinal extremity (72a) of the release paper (22) being applied onto a first portion of the second sub-area (40c) of adhesive;
- folding the front portion (F) onto the central portion (C) and folding the rear portion (R) onto the front portion (F) or folding the rear portion (R) onto the central portion (C) and folding the front portion (F) onto the rear portion (R);
- optionally folding the wings (24) onto the folded front and rear portions (F,R);
- wrapping the release paper (22) around the folded absorbent article (10) onto the folded front and rear portion (F,R) or optionally onto the folded wings (24); and
- securing the release paper (22) to the second portion of the second sub-area (40c) of adhesive with the second longitudinal extremity (72b) of the release paper (22) being applied onto the second portion of the second sub-area (40c) of adhesive.

11. Method for folding an absorbent article (10) according to the claims 2 to 9, wherein said method comprises the following steps :
- providing an absorbent article 10 and said release paper (22);
- optionally folding the wings (24) onto the central portion (C) and applying a release liner (47) to keep the wings folded onto the central portion (C);
- folding the front portion (F) onto the central portion (C);
- applying a release paper (22) in a direction orthogonal to the longitudinal direction of the absorbent article (10) with a first longitudinal extremity (72a) of the release paper (22) being applied onto the first sub-area (40f) of adhesive;
- folding the rear portion (R) onto the release paper (22);
- wrapping the release paper (22) around the folded absorbent article (10) and securing the release paper (22) onto the second sub-area (40c) of adhesive; and
- securing the release paper (22) to the third sub-area (40r) of adhesive with the second longitudinal extremity (72b) of the release paper (22) being applied onto the third sub-area (40r) of adhesive.

12. Method according to claim 10 or 11, wherein the absorbent article (10) comprises wings (24), said method comprises a further step of applying a release liner (47) onto second area of adhesive (45) arranged on the wings (24).

13. A container comprising a plurality of assemblies (1) according to any of the claims 1 to 9, said container being selected from a reusable bag, a paper bag, a cardboard box or a metal box.
